**Europäisches Patentamt**

**European Patent Office**

**Office européen des brevets**

(19)

(11) Veröffentlichungsnummer : **0 418 796 B1**

(12) **EUROPÄISCHE PATENTSCHRIFT**

(45) Veröffentlichungstag der Patentschrift :
**26.01.94 Patentblatt 94/04**

(51) Int. Cl.⁵ : **C12N 5/00**

(21) Anmeldenummer : **90117893.9**

(22) Anmeldetag : **18.09.90**

(54) **Verfahren zur Kultivierung von Zellen in Mikrohohlkugeln.**

(30) Priorität : **21.09.89 DE 3931433**

(43) Veröffentlichungstag der Anmeldung :
**27.03.91 Patentblatt 91/13**

(45) Bekanntmachung des Hinweises auf die
Patenterteilung :
**26.01.94 Patentblatt 94/04**

(84) Benannte Vertragsstaaten :
**AT CH DE DK FR GB IT LI NL SE**

(56) Entgegenhaltungen :
**EP-A- 0 313 008
WO-A-87/00197
CHEMICAL ABSTRACTS, Band 111, Nr. 5, 31.
Juli 1989, Seite 482, ZusammenfassungNr.
37932s, Columbus, Ohio, US; A.D. MURDIN et
al.: "Growth and metabolism ofhybridomas
immobilized in packed beds: comparison with
static and suspensioncultures", & ENZYME
MICROB. TECHNOL. 1989, 11(6), 341-6**

(73) Patentinhaber : **HOECHST
AKTIENGESELLSCHAFT
D-65926 Frankfurt (DE)**

(72) Erfinder : **Buchholz, Rainer, Dr.
Grosser Ring 38
W-5239 Unnau (DE)**
Erfinder : **Giani, Carlo, Dr.
Hedderichstrasse 69
W-6000 Frankfurt am Main (DE)**
Erfinder : **Fricke, Ulrich
Am Flachsland 56
W-6233 Kelkheim/Taunus (DE)**
Erfinder : **Rüppel, Dieter, Dr.
Karl-König-Weg 1
W-6230 Frankfurt am Main (DE)**
Erfinder : **Walch, Axel, Dr.
Hans-Sachs-Strasse 5
W-6000 Frankfurt am Main (DE)**
Erfinder : **Bayer, Thomas, Dr.
Bismarckstrasse 14
W-6232 Bad Soden am Taunus (DE)**
Erfinder : **Kurrle, Roland, Dr.
Kiefernweg 12
W-3556 Niederweimar (DE)**
Erfinder : **Krause, Dieter
Bahnhofstrasse 9
W-6301 Heuchelheim (DE)**
Erfinder : **Lang, Wiegand
Akazienweg 1
W-3551 Cölbe (DE)**
Erfinder : **Reiche, Asmus, Dr.
Auf der Hube 9
W-3550 Marburg (DE)**

EP 0 418 796 B1

Jouve, 18, rue Saint-Denis, 75001 PARIS

## Beschreibung

Die Erfindung betrifft ein Verfahren zur Kultivierung von Zellen in Mikrohohlkugeln, die in einem Festbett vorliegen und diskontinuierlich oder kontinuierlich mit Nährstoffen versorgt werden.

Es sind eine Reihe verschiedener Methoden bekannt, mit denen ganze Zellen immobilisiert werden können. So können Zellen durch geeignete Maßnahmen in einem vernetzten Gel eingeschlossen werden und bleiben darin weiterhin lebensfähig und aktiv (Klein, Wagner "Methods for the Immobilisation of Microbial Cells" in Appl. Biochem. Bioeng. 4: 11-51 (1983)). In der Praxis hat es sich jedoch gezeigt, daß lebende Zellen die Gelmatrix durch Wachstum sprengen und dann in die umgebende Nährlösung gelangen können. Dort können sie sich weiter vermehren und dadurch die Zellrückhaltung in kontinuierlichen Verfahren sehr erschweren.

In der US Patentschrift 2 958 517 wird eine Vorrichtung zur Kultivierung von freien Säugetierzellen offenbart, in der die Durchmischung der Nährlösung mit einem magnetisch angetriebenen Rührstab erfolgt. Bei dieser Verfahrensweise kommt es jedoch immer zu Kollisionen der Zellen untereinander und daraus folgend zu einer Beeinträchtigung der Lebensfunktionen, die zum Absterben der Zellen führen kann.

In US 706 872 wird eine kontinuierliche Kultivierung von Säugetierzellen auf porösen schwammartigen Partikeln beschrieben. Etwa 1 bis 5 % der Zellpopulation im Reaktor kommen frei in der Nährlösung vor, dadurch können die Rückhaltevorrichtungen für die Immobilisate wie Membranen oder Glassinterplatten durch die freien Zellen leicht belegt und verstopft werden.

Insbesondere die Züchtung von pflanzlichen oder tierischen Zellen ist sehr schwierig. Diese Zellen haben sehr empfindliche und zerbrechliche Membranen, die schon leicht durch geringe mechanische Einwirkung geschwächt oder zerstört werden können. Dadurch werden die Lebensfähigkeit und die Produktivität dieser Zellen stark beeinträchtigt.

Eine andere Methode der Immobilisierung stellt die Technik des Einschlusses von Zellen in semipermeable Membranen dar, die im weiteren als Mikroholkugeln bezeichnet werden. In der Europäischen Patentanmeldung 0 173 915 oder 0 280 155 und in der Deutschen Offenlegungsschrift 3 529 203 werden Beispiele für diese Technik genannt. In der Deutschen Offenlegungsschrift wird außerdem gezeigt, wie immobilisierte Zellen in einem gerührten Reaktor gezüchtet werden können. Aber auch in diesem Reaktor kommt es noch zu starken mechanischen Belastungen der Zellen durch den Rührer. Es kann zur Zerstörung der Kapseln kommen und zum Freisetzung der eingeschlossenen Zellen und letztlich zu einer Schädigung der Zellen.

Überraschender Weise wurde nun gefunden, daß durch die dichte Anordnung von Mikrohohlkugeln in einem Festbett, die oben genannten Schwierigkeiten überwunden werden können. Trotz der dichten Packung behalten die Mikrohohlkugeln ihre Stabilität und ermöglichen so den sie enthaltenen Zellen optimale Wachstums- und Produktionsbedingungen. Diese enge Anordnung der Mikrohohlkugeln im Reaktor verhindert überraschender Weise auch eine Kanalbildung zwischen den Mikrohohlkugeln. Dadurch wird eine gleichmäßige, gut zu kontrollierende Versorgung der Zellen mit allen benötigten Nährstoffen sichergestellt. Dies ermöglicht damit auch eine lange, über viele Generationen reichende, störungsfreie Kultivierung der Zellen.

Die Erfindung betrifft somit ein Verfahren zur Kultivierung von Zellen in Mikrohohlkugeln, deren Membran aus einem anionischen Polysaccharidgel oder aus einer Polyelektrolytmembran besteht, das dadurch gekennzeichnet ist, daß die Mikrohohlkugeln in einem Festbett vorliegen.

Die Mikrohohlkugeln liegen im Festbett als dichte Kugelpackung im Nährlösungsstrom. Sie erfahren nur geringe räumliche Lageveränderungen und damit treten nur geringe mechanische Reibungen, die zur Zerstörung führen könnten, auf den Membranen der Mikrohohlkugeln auf.

Die Erfindung wird im folgenden detailliert beschrieben, insbesondere in ihren bevorzugten Ausführungsformen. Ferner wird die Erfindung in den Ansprüchen definiert.

Die für die Erfindung geeigneten Mikrohohlkugeln bestehen aus einer biokompatiblen, nicht toxischen, semipermeablen, wasserunlöslichen Membran. Die Herstellung solcher Membranen wird z.B. in der Europäischen Patentanmeldung 0 173 915 beschrieben. Dazu werden die Zellen insbesondere in einer wäßrigen Lösung des Kernpolymers suspendiert. Das Kernpolymer bewirkt eine Viskositätserhöhung der Zellsuspension, so daß beim nachfolgenden Eintropfen in die anionische Polysaccharidlösung eine Durchmischung der Lösungen verhindert wird. Als Kernpolymer sind alle neutralen, wasserlöslichen, biologisch verträglichen Polymere geeignet, die die Viskosität erhöhen. Es sind dies z.B. Hydroxypropylmethylcellulose oder Hydroxymethylcellulose. Diese Kernpolymere werden mit einem oder mehreren zweiwertigen Kationen, wie z.B. $CaCl_2$, versetzt. Dann wird die Mischung in die Tropfenform übergeführt und in eine anionische Polysaccharidlösung, aus z.B. Alginat, Carrageenan, Chitosan, Pektinat oder Carboxymethylcellulose, gebracht. Bevorzugt wird Alginat verwendet. An der Phasengrenze zwischen Kernpolymer und Polysaccharidlösung wird durch die Anwesenheit einer oder mehrerer zweiwertiger Kationen eine semipermeable Membran gebildet, die dann die Zellen umschließt. Die Membran besteht aus dem anionischen Polysaccharid, das durch die zweiwertigen Kationen in die Gelform übergeführt wird. Das Kernpolymer, in dem die Zellen suspendiert sind, bleibt hingegen flüssig.

Auf analoge Weise können Zellen, wie in der Europäischen Patentanmeldung 0 280 155 beschrieben, auch in einer wäßrigen Lösung eines anionischen Polymers (Polysäure), wie z.B. Alginat, Carrageenan, Hyaluronsäure, Carboxymethylcellulose, Xanthan oder Furcellaran, suspendiert, in Tropfenform übergeführt und anschließend in eine wäßrige Lösung eines kationischen Polymers (Polybase), wie z.B. eines Copolymerisats aus 1-Vinyl-3-methylimidazoliumchlorid und 1-Vinyl-2-pyrrolidon oder eines Polyallylamin-2-hydroxy-propylen-Copolymerisats, gebracht werden. Bevorzugt wird ein Copolymerisat aus 1-Vinyl-3-methylimidazoliumchlorid und 1-Vinyl-2-pyrrolidon verwendet. An der Phasengrenze zwischen Polysäure und Polybase bildet sich eine semipermeable Polyelektrolytmembran aus, die die Zellen einschließt. Auch in diesem Fall bleibt das Kernpolymer, in dem die Zellen suspendiert sind, flüssig. Die Membran verhindert den Durchtritt der Zellen, ist aber frei permeabel für Gase und Mediumsbestandteile.

In diesen Mikrohohlkugeln können alle lebensfähigen Zellen eingeschlossen und kultiviert werden. Es sind dies Bakterien, Pilze oder Hefen und insbesondere alle Zellinien tierischen oder pflanzlichen Ursprungs, sowie besonders bevorzugt Hybridomazellen. Unter geeigneten Bedingungen vermehren sich die Zellen durch Zellteilung in den Mikrohohlkugeln. Durch die semipermeable Membran hindurch erfolgt durch Diffusion die Versorgung mit Nährstoffen und der Abtransport von gebildeten Produkten.

Die Kultivierung erfolgt in einem Gefäß, in dem die Mikrohohlkugeln in einer dichten Packung vorliegen. Das Gefäß kann aus inertem Material bestehen, wie z.B. Metall, Keramik, Glas oder einem Kunststoffmaterial, das durch chemische oder physikalische Methoden sterilisiert werden kann. Das Gefäß kann insbesondere in einer zylinderähnlichen Form vorliegen. Das Verhältnis von Höhe zu Durchmesser kann in einem weiten Bereich schwanken. Das Verhältnis von Höhe zu Breite kann im Bereich von 100:1 bis 1:100, insbesondere von 2:1 bis 20:1, variiert werden.

Das Kultivierungsgefäß besitzt an entgegengesetzten Enden Einlaß- und Auslaßöffnungen; dazwischen befinden sich die Mikrohohlkugeln. Die Öffnungen sind so angeordnet, daß die Nährlösung die gesamten dazwischenliegenden Mikrohohlkugeln durchströmen kann. Zwischen den Öffnungen und den Mikrohohlkugeln liegen Rückhaltevorrichtungen für die Mikrohohlkugeln, wie z.B. Membranen oder Glassinterplatten, die aber für die Nährlösung leicht durchlässig sind. Mit einer solchen Anordnung ist es möglich, Nährlösung kontinuierlich oder diskontinuierlich durch das Gefäß mit jeder gewünschten Rate fließen zu lassen. Das Flüssigkeitsvolumen bleibt in dieser Zeit konstant. Durch eine entsprechende Vorrichtung kann die Nährlösung in einer Schleife auch zyklisiert werden, so daß die oft teuren Nährstoffbestandteile besser ausgenutzt werden können.

Alle Regel-, Meß- und Einstellvorgänge können, wenn notwendig außerhalb des Kultivierungsgefäßes erfolgen. Es sind dies vorallem die Messung der Temperatur, des pH-Werts und des Sauerstoffpartialdrucks, aber auch der Gasaustausch von $O_2$ und $CO_2$, die Regulierung des pH-Werts und das Erneuern von Nährlösungsbestandteilen.

Ohne Beeinträchtigung der eingekapselten Zellen kann aus der Nährlösung ein Teil abgezweigt werden, um die hergestellten Produkte abzutrennen und aufzuarbeiten. Die abgezweigte Nährlösung kann auch wieder durch frische ersetzt werden. Der Nährstoffaustausch und die Produktgewinnung kann diskontinuierlich oder kontinuierlich aus der Recyclingvorrichtung des Nährstoffstroms erfolgen.

Die Wahl und Einstellung der chemischen und physikalischen Kultivierungsparameter erfolgt entsprechend der Wachstums- und Produktionsbedürfnisse der in den Mikrohohlkugeln eingeschlossenen Zellen. Die Grenzen dieser Kultivierungsparameter können in weiten Bereichen schwanken. Einem Fachmann fällt es aber nicht schwer die jeweils optimalen Parameter für die verwendeten Zellen herauszufinden.

Für die Hybridomazellen haben sich beispielsweise folgende physikalischen und chemischen Kultivierungsparameter bewährt. Die Kultivierungstemperatur liegt zwischen 35 und 39°C, bevorzugt bei 37°C. Ausreichendes Wachstum erzielt man bei einem pH-Wert zwischen 6,8 und 7,2, bevorzugt bei 7,0. Die Flußrate durch den Reaktor kann je nach Wachstumsstadium in einem weiten Bereich schwanken. Gute Ergebnisse erhält man zwischen 0,1 und 50 Liter Medium je Stunde und Liter Reaktorvolumen, bevorzugt zwischen 5 und 20 l/h·l.

Überraschend wurde gefunden, daß die Mikrohohlkugeln trotz der dichten Packung in dem Kultivierungsgefäß ihre Stabilität behielten. Es kam zu keiner Kanalbildung und zu keinem Verklumpen der Kapseln. Die Kultivierung konnte so über lange Zeit durchgeführt werden. Durch die dichte Packung der Mikrohohlkugeln und das ständige Nährstoffrecycling, ist es möglich, auf sehr kleinem Raum unter sterilen Bedingungen mit einer einfachen Apparatur Zellen zu kultivieren und Produkte herzustellen. Insbesondere das Nährstoffrecycling führt zu einer effektiven und ökonomischen Ausnutzung der benötigten oft sehr teuren Nährstoffe. Auch die Regeneration der Nährlösung ist durch die selektive Abtrennung von Stoffwechselendprodukten, wie z.B. Milchsäure, $CO_2$ oder Ammoniumionen, durch Ionenaustauscher oder andere Trennverfahren im zellfreien Nährstoffstrom möglich.

Die im folgenden aufgeführten Beispiele dienen der weiteren Erläuterung der Erfindung.

**Beispiel 1**

Eine Suspension von Hybridomazellen wird mit einer 3 %igen Carrageenanlösung (Sigma Chemie GmbH, München) in Dulbecco's Medium (Dulbecco u. Freeman, (1959), Virology 8, 396) 1:2 (Massenverhältnis) verdünnt. Die Lösung wird über eine Düse vertropft. Die Düse besteht aus einer Kanüle, Innendurchmesser 0,2 mm, Außendurchmesser 0,4 mm. Sie ist konzentrisch in einem Hohlzylinder eingelassen, so daß über den entstehenden Ringspalt ein tangentialer Luftstrom erzeugt werden kann, der die aus der Kanüle austretenden Tropfen abdrückt. Je nach Luftgeschwindigkeit betrugen die Tropfendurchmesser 0,1 mm - 3 mm. Die Tropfen fielen in eine Lösung der Polybase.

Die Polybase wird folgendermaßen hergestellt: In einem 4 Liter Glaskolben werden 1443 g (10 Mol) 1-Vinyl-3-methylimidazoliumchlorid und 56 g (0,5 Mol) 1-Vinyl-2-pyrrolidon in 3,8 l Wasser gelöst, das 38 g Kaliumperoxodisulfat als Initiator enthält. Der Ansatz wird 6 h bei 60°C unter Stickstoff polymerisiert. Man erhält eine klare gelb-braune 40 %ige Lösung mit neutralem pH-Wert. 100 ml der 40 %igen Lösung werden auf 2 l mit einer 0,9 %igen wäßrigen NaCl-Lösung aufgefüllt. Etwa 100-120 ml Carrageenan/Zellsuspension werden in 1 Liter der verdünnten Polymerisatlösung getropft.

Nach der Verdünnung läßt man die Kapseln sedimentieren, dekantiert die Lösung ab und suspendiert 3 mal mit 0,9 % NaCl-Lösung. Anschließend können Zellen in den Mikrohohlkugeln kultiviert werden.

**Beispiel 2**

28,2 g Hydroxypropyl-methylzellulose wird in 675 ml 0,9 %iger NaCl-Lösung aufgelöst. Danach werden 75 ml einer 13,3 %igen $CaCl_2$ x $2H_2O$-Lösung zugegeben. Diese Lösung wird mit der Hybridomazellsuspension im Verhältnis 3:1 (Massenverhältnis) gemischt. Diese Suspension wird wie in Beispiel 1 beschrieben vertropft. Die Tropfen werden in einer Alginatlösung aufgefangen. Die Alginatlösung wird folgendermaßen hergestellt. 15 g Alginat werden in 500 ml 0,9 %iger NaCl-Lösung gelöst und anschließend mit 0,9 %iger NaCl-Lösung auf 2 l verdünnt. Es werden 80 ml der obigen Zellsuspension in 1 l Alginatlösung eingetropft. An der Phasengrenze zwischen Tropfen und Alginatlösung polymerisiert ein Alginatgel aus, daß die Zellen eingeschlossen hält. Die Mikrohohlkugeln werden 3 mal mit 0,9 %iger NaCl-Lösung gewaschen, dann in eine 2 %ige $CaCl_2$ x $2H_2O$-Lösung überführt und 2 min gerührt. Anschließend wird die Lösung dekantiert und die Mikrohohlkugeln mehrfach mit 0,9 %iger NaCl-Lösung gewaschen.

**Beispiel 3**

Die nach Beispiel 2 eingekapselten Zellen werden unter sterilen Bedingungen in eine sterile Reaktorsäule überführt. Die Säule wird vollständig mit Kapseln angefüllt und dann in einem geschlossenen System ständig mit Medium überströmt. Die Einstellung und Regelung der Parameter erfolgt in einem 5 l Reaktor, der über Leitungen mit der Säule verbunden ist. Der Gasautausch wird im Reaktor durch Schlauchmembranen bewirkt. Es wird unter folgenden Bedingungen kultiviert:

```
Medium              Dulbecco's-Nährlösung (Dulbecco (1959),
                                          Virology, 8, 296)

                    10 % foetales Kälberserum
Volumen der Säule   0,4 l
Mediumsfluß           7   l/h
pH                  7,0
pO2                 60 % Luftsättigung
T                   37°C
```

Der $pO_2$-Gehalt wird am Säulenende mit einer Sauerstoffelektrode gemessen. Die Überströmung wird mit einer Schlauchpumpe gewährleistet. Die Versuchszeit erstreckt sich über 25 Tage. Das Medium wird 4 mal gewechselt. Dazu wurde jeweils 50 % des alten Mediums durch neues ersetzt. Der Zeitpunkt des Mediumswechsels wird durch die Messung von Stoffwechselprodukten bestimmt. Bei Ammoniumkonzentrationen von mehr als 3 mmol/l und Glutaminmengen von weniger als 1,5 mmol/l erfolgt ein Mediumswechsel. Die Zellzahl beträgt am Anfang 20.500 Zellen je Kapsel und steigt am Ende der Kultivierung auf etwa 200.000 Zellen je Kapsel an.

Die mittlere Antikörperproduktion beträgt 0,08 mg/ml Reaktorvolumen und Tag.

Die Bestimmung von Ammonium- und Glutaminmenge erfolgte mit enzymatischer Standardbestimmung der Firma Boehringer, Mannheim.

**Beispiel 4**

Die Kultivierung der in Mikrohohlkapseln eingeschlossenen Zellen erfolgt wie in Beispiel 3 angegeben. Als Reaktor wird eine zylindrische Glassäule mit 400 ml Nutzvolumen verwendet. Das Verhältnis von Höhe zu Durchmesser beträgt 3 zu 1. An Ein- und Ausgang der Glassäule werden Glassinterplatten mit einem Porendurchmesser von 0,1 mm zur Rückhaltung der Mikrohohlkapseln installiert. Die Fläche der Glassinterplatte am Ausgang ist doppelt so groß wie am Eingang.

**Beispiel 5**

Die nach Beispiel 1 eingekapselten Zellen werden unter sterilen Bedingungen in eine wie in Beispiel 3 beschriebenen sterile Reaktorsäule überführt. Die Kultivierung erfolgt wie in Beispiel 3 angegeben, allerdings in Iscove's Nährlösung (Iscove u. Melchers (1978), J. Exp. Med., 147: 923). Die mittlere Antikörperproduktion beträgt 0,06 mg/ml Reaktorvolumen und Tag. Die Kultivierung erstreckte sich über 4 Wochen.

**Patentansprüche**

1. Verfahren zur Kultivierung von Zellen in Mikrohohlkugeln, deren Membran aus einem anionischen Polysaccharidgel oder aus einer Polyelektrolytmembran besteht, dadurch gekennzeichnet, daß die Mikrohohlkugeln in einem Festbett vorliegen.

2. Verfahren nach Anspruch 1, dadurch gekennzeichnet, daß Zellen pflanzlichen oder tierischen Ursprungs kultiviert werden.

3. Verfahren nach Anspruch 2, dadurch gekennzeichnet, daß Hybridomazellen kultiviert werden.

4. Verfahren nach einem oder mehreren der Ansprüche 1 bis 3, dadurch gekennzeichnet, daß die Membran der Mikrohohlkugeln aus einem Alginatgel besteht.

5. Verfahren nach Anspruch 4, dadurch gekennzeichnet, daß die Bildung der Alginatmembran durch Komplexierung mit einem Vernetzungsmittel aus der Tropfenphase erfolgt.

6. Verfahren nach einem oder mehreren der Ansprüche 1 bis 3, dadurch gekennzeichnet, daß die Membran der Mikrohohlkugeln aus einem Copolymerisat, bestehend aus 1-Vinyl-3-methylimidazoliumchlorid und 1- Vinyl-2-pyrrolidon, besteht.

7. Verfahren nach einem oder mehreren der Ansprüche 1 bis 6, dadurch gekennzeichnet, daß die Nährstoffversorgung durch einen geschlossenen Nährlösungsstrom erfolgt.

8. Verfahren nach Anspruch 7, dadurch gekennzeichnet, daß die Nährstoffversorgung diskontinuierlich oder kontinuierlich erfolgt.

9. Verfahren nach einem oder mehreren der Ansprüche 1 bis 7, dadurch gekennzeichnet, daß der Nährstoffaustausch diskontinuierlich oder kontinuierlich erfolgt.

**Claims**

1. A method for cultivating cells in microporous beads whose membrane is composed of an anionic polysaccharide gel or of a polyelectrolyte membrane, wherein the microporous beads are located in a fixed bed.

2. The method as claimed in claim 1, wherein cells of plant or animal origin are cultivated.

3. The method as claimed in claim 2, wherein hybridoma cells are cultivated.

4. The method as claimed in one or more of claims 1 to 3, wherein the membrane of the microporous beads is composed of an alginate gel.

5. The method as claimed in claim 4, wherein the alginate membrane is formed by complexation with a crosslinking agent from the drop phase.

6. The method as claimed in one or more of claims 1 to 3, wherein the membrane of the microporous beads is composed of a copolymer composed of 1-vinyl-3-methylimidazolium chloride and 1-vinyl-2-pyrrolidone.

7. The method as claimed in one or more of claims 1 to 6, wherein the nutrient supply is effected by a closed flow of nutrient solution.

8. The method as claimed in claim 7, wherein the nutrient supply takes place discontinuously or continuously.

9. The method as claimed in one or more of claims 1 to 7, wherein the nutrient replacement takes place discontinuously or continuously.

**Revendications**

1. Procédé pour cultiver des cellules dans des microparticules dont la membrane est constituée d'un gel anionique de polysaccharide ou d'une membrane de polyélectrolyte, caractérisé en ce que les microparticules se trouvent dans un lit fixe.

2. Procédé selon la revendication 1, caractérisé en ce que l'on cultive des cellules d'origine végétale ou animale.

3. Procédé selon la revendication 2, caractérisé en ce que l'on cultive des cellules d'hybridome.

4. Procédé selon une ou plusieurs des revendications 1 à 3, caractérisé en ce que la membrane des microparticules est constituée d'un gel d'alginate.

5. Procédé selon la revendication 4, caractérisé en ce que la membrane d'alginate est formée par complexation à l'aide d'un agent réticulant provenant de la phase existant sous forme de gouttes.

6. Procédé selon une ou plusieurs des revendications 1 à 3, caractérisé en ce que la membrane des microparticules est constituée d'un copolymérisat de chlorure de 1-vinyl-3-méthylimidazolium et de 1-vinyl-2-pyrrolidone.

7. Procédé selon une ou plusieurs des revendications 1 à 6, caractérisé en ce que la distribution de substance nutritive est assurée par une solution nutritive s'écoulant en circuit fermé.

8. Procédé selon la revendication 7, caractérisé en ce que la distribution de substance nutritive est effectuée en continu ou de façon discontinue.

9. Procédé selon une ou plusieurs des revendications 1 à 7, caractérisé en ce que le renouvellement de substance nutritive est effectué en continu ou de façon discontinue.